# EUROPEAN PATENT APPLICATION

(11) **EP 4 534 440 A1**
(43) Date of publication of application: **09.04.2025**
(21) Application number: 23811762.6
(22) Date of filing: 22.05.2023
(51) Int. Cl.: B65D 83/00

(54) **STORAGE CONTAINER ADAPTER, STORAGE CONTAINER, AND STORAGE DEVICE**

(30) Priority: 24.05.2022 JP 2022084753
(71) Applicant: Musashi Engineering, Inc., Mitaka-shi, Tokyo 181-0011 (JP)
(72) Inventor: IKUSHIMA, Kazumasa, Mitaka-shi, Tokyo 181-0011 (JP)
(74) Representative: TBK
(86) International application number: PCT/JP2023/018859
(87) International publication number: WO 2023/228890

(57) **Abstract**

Problem: To provide a reservoir container adapter, a reservoir container, and a reservoir device that make it possible to confirm that the reservoir container and the reservoir container adapter are in a right connection position even if some dimensional difference occurs due to uneven shrinkage during manufacturing.

Solution: A reservoir container adapter 10 that is attached to a reservoir container 20 including a reservoir cylinder 21 and a pair of brims 23 includes a base 11, a blocking portion 13, a pair of lateral members 12, and a pair of brim-holding claws 14. The pair of brim-holding claws 14 are provided with a first engagement element 15 and the pair of brims are provided with second engagement elements 27 that engage with the first engagement element 15. When the blocking portion 13 is inserted into an upper opening of the reservoir container 20 and rotation in one direction is performed, the brims 23 enter gaps between the brim-holding claws 14 and the base 11 and the first engagement element 15 and the second engagement element 27 engage with each other to perform positioning.

## Description

### Technical Field

The present invention relates to a reservoir container adapter that is attached to a reservoir container used for a liquid material discharge device, the reservoir container, and a reservoir device.

### Background Art

A liquid material discharge device includes a reservoir container in which a liquid material to be discharged is stored. An adapter having a joint for connecting to a pressurized-air source is attached to the reservoir container, and the liquid material is transferred to a nozzle by pressurized air being supplied into the reservoir container through the joint. For example, Patent Document 1 discloses a reservoir barrel including a pair of ears, and an adapter including: a first end; a second end that is coupled to a source of pressurized fluid; a first and second hubs for engaging with the ears on the barrel; and a seal extending from the first end and configured to sealingly engage with an open end of the barrel. A protruding detent extending radially outward is formed on a side end of an ear on the barrel in Patent Document 1, and the adapter is securely fixed by the protruding detent engaging with a slot provided in an inner wall of the adapter.

When the reservoir container is not connected to a discharge device, a closure lid is put on it for storage or transportation. The closure lid includes engagement claws for engaging with the ears on the barrel, and a sealing portion that sealingly engages with the open end of the barrel (see, for example, Fig. 4 of Patent Document 1, and Patent Document 2). The closure lid, which is used for sealing the inside of the reservoir container, is not provided with the joint for connecting to the pressurized-air source like the reservoir container adapter described above. Since the closure lid is not intended to be used while the inside of the reservoir container is under pressure, its holding strength is weaker than that of the reservoir container adapter. In contrast, the reservoir container adapter described above is designed on the assumption that it is used while pressurized air is applied to the inside of the reservoir container, and is thus manufactured to be thicker and sturdier than the closure lid in order to prevent air leakage due to deformation.

### Prior Art List

### Patent Document

Patent Document 1: Japanese Patent Publication No. 5551433
Patent Document 2: Japanese Patent Publication No. 4986600

### Summary of the Invention

### Problems to be Solved by the Invention

Although the reservoir container and the reservoir container adapter described above are manufactured generally by resin molding, dimensional control at the time of manufacturing is extremely difficult due to uneven shrinkage during cooling in a molding process. Particularly, the reservoir container adapter that is manufactured to be thicker than the reservoir container is subject to larger dimensional difference caused by the issue of uneven shrinkage.

In a case where a structure in which accurate dimensions are required is adopted for both a reservoir container and a reservoir container adapter, the issue of dimensional difference becomes more significant. For example, in Patent Document 1, if the protrusion provided on the reservoir container is manufactured larger than specified and the slot provided in the inner wall of the reservoir container adapter is manufactured smaller than specified, there arises an issue that the fit is tight or an issue that rotation stops before the protrusion fits into the slot. Conversely, if the protrusion provided on the reservoir container is manufactured smaller than specified and the slot provided in the reservoir container adapter is manufactured larger than specified, there arises an issue that the protrusion does not fit in the slot and the fixation is insufficient. In addition, if a pair of the slots provided in the inner wall of the reservoir container adapter are manufactured with the distance dimension therebetween shorter than specified, there arises an issue that the fit is tight (or large dimensional difference may stop rotation before the protrusions fit into the slots), and if a pair of the slots are manufactured with the distance dimension therebetween longer than specified, there arises an issue that the fit is loose. This distance dimension between the slots is affected by uneven shrinkage in the longitudinal direction of the main body that is the longest portion in the reservoir container adapter, and thus it is more difficult to control dimensional quality than in other portions.

As the above issues of dimensional difference cause fit feeling to vary for each combination of a reservoir container and a reservoir container adapter, it is in some cases difficult to confirm that the reservoir container and the reservoir container adapter are in a right connection position.

Therefore, an object of the present invention is to provide a reservoir container adapter, a reservoir container, and a reservoir device that can solve the above-described problem.

### Means for Solving the Problems

A reservoir device according to the present invention is a reservoir device including a reservoir container and an adapter configured to be attached to the reservoir container, wherein the reservoir container includes a reservoir cylinder having an upper opening and a lower opening, and a pair of brims extending laterally from an upper end of the reservoir cylinder, the adapter includes a base configured to be connected to an air pipe, a blocking portion extending downward from a central portion of the base, and a pair of brim-holding portions provided on opposite short sides of the base and configured to hold the pair of brims, the pair of brim-holding portions include a pair of lateral members extending downward from the base, and a pair of brim-holding claws extending toward the blocking portion from the pair of lateral members, an upper surface of at least one of the pair of brim-holding claws or a lower surface of the base is provided with a first engagement element, and a lower surface or an upper surface of each of the pair of brims is provided with a second engagement element configured to engage with the first engagement element, wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

In the reservoir device, a distance W1 between inner walls of the pair of lateral members may be larger than a distance W2 between most protruded points of the pair of brims.

In the reservoir device, the base or the brim-holding claws may include a window provided at a position facing the first engagement element.

In the reservoir device, the pair of lateral members may include rotation stop surfaces that come in contact with short sides of the pair of brims in such a position that the first engagement element and the second engagement element engage with each other.

In the reservoir device, the pair of brim-holding claws may include tapered surfaces for the brims to come in contact with when entering the gaps between the brim-holding claws and the base.

In the reservoir device, the gaps between the brim-holding claws and the base may have such a size that the holding claws elastically deform when the brims enter the gaps.

In the reservoir device, the adapter may be made of resin.

In the reservoir device, the reservoir container may be made of resin that is transparent, and the adapter may be made of resin that is colored.

In the reservoir device, the adapter may be made of material harder than material of the brims.

In the reservoir device, the first engagement element may include a protruded portion provided on an upper surface of each of the pair of brim-holding claws, and the second engagement element may include a recessed portion provided in a lower surface of each of the pair of brims.

In the reservoir device, the first engagement element may include a recessed portion provided in an upper surface of each of the pair of brim-holding claws, and the second engagement element may include a protruded portion provided on a lower surface of each of the pair of brims.

In the reservoir device, the first engagement element may include a protruded portion provided on a lower surface of the base, and the second engagement element may include a recessed portion provided in an upper surface of each of the pair of brims.

In the reservoir device, the first engagement element may include a recessed portion provided in a lower surface of the base, and the second engagement element may include a protruded portion provided on an upper surface of each of the pair of brims.

In the reservoir device, when the first engagement element is a protruded portion and the second engagement element is a recessed portion, the first engagement element and the second engagement element may engage with each other at a position closer to a position furthest from an entrance start position for the brims in the gaps than a midpoint between the entrance start position for the brims in the gaps and the position furthest from the entrance start position for the brims in the gaps.

In the reservoir device, when the first engagement element is a recessed portion and the second engagement element is a protruded portion, the first engagement element and the second engagement element may engage with each other at a position closer to an entrance start position for the brims in the gaps than a midpoint between the entrance start position for the brims in the gaps and a position furthest from the entrance start position for the brims in the gaps.

A reservoir container adapter according to a first aspect of the present invention is a reservoir container adapter used for the reservoir device, the reservoir container adapter including a base configured to be connected to an air pipe, a blocking portion extending downward from a central portion of the base, and a pair of brim-holding portions provided on opposite short sides of the base and configured to hold the pair of brims, wherein the first engagement element includes a protruded portion provided on an upper surface of each of the pair of brim-holding claws, wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

A reservoir container adapter according to a second aspect of the present invention is a reservoir container adapter used for the reservoir device, the reservoir container adapter including a base configured to be connected to an air pipe, a blocking portion extending downward from a central portion of the base, and a pair of brim-holding portions provided on opposite short sides of the base and configured to hold the pair of brims, wherein the first engagement element includes a recessed portion provided in an upper surface of each of the pair of brim-holding claws, wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

A reservoir container adapter according to a third aspect of the present invention is a reservoir container adapter used for the reservoir device, the reservoir container adapter including a base configured to be connected to an air pipe, a blocking portion extending downward from a central portion of the base, and a pair of brim-holding portions provided on opposite short sides of the base and configured to hold the pair of brims, wherein the first engagement element includes a protruded portion provided on a lower surface of the base, wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

A reservoir device according to a fourth aspect of the present invention is a reservoir container adapter used for the reservoir device, the reservoir container adapter including a base configured to be connected to an air pipe, a blocking portion extending downward from a central portion of the base, and a pair of brim-holding portions provided on opposite short sides of the base and configured to hold the pair of brims, wherein the first engagement element includes a recessed portion provided in a lower surface of the base, wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

A reservoir container according to a first aspect of the present invention is a reservoir container used for the reservoir device, the reservoir container including a reservoir cylinder having an upper opening and a lower opening, and a pair of brims extending laterally from an upper end of the reservoir cylinder, wherein the second engagement element includes a recessed portion provided in a lower surface of each of the pair of brims, wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

A reservoir container according to a second aspect of the present invention is a reservoir container used for the reservoir device, the reservoir container including a reservoir cylinder having an upper opening and a lower opening, and a pair of brims extending laterally from an upper end of the reservoir cylinder, wherein the second engagement element includes a protruded portion provided on a lower surface of each of the pair of brims, wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

A reservoir container according to a third aspect of the present invention is a reservoir container used for the reservoir device, the reservoir container including a reservoir cylinder having an upper opening and a lower opening, and a pair of brims extending laterally from an upper end of the reservoir cylinder, wherein the second engagement element includes a recessed portion provided in an upper surface of each of the pair of brims, wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

A reservoir container according to a fourth aspect of the present invention is a reservoir container used for the reservoir device, the reservoir container including a reservoir cylinder having an upper opening and a lower opening, and a pair of brims extending laterally from an upper end of the reservoir cylinder, wherein the second engagement element includes a protruded portion provided on an upper surface of each of the pair of brims, wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

### Advantageous Effect of the Invention

According to the present invention, it is possible to provide a reservoir container adapter, a reservoir container, and a reservoir device that make it possible to confirm that the reservoir container and the reservoir container adapter are in a right connection position even if some dimensional difference occurs due to uneven shrinkage during manufacturing.

### Brief Description of the Drawings

[Fig. 1] Fig. 1 is a partial side cross-sectional view illustrating a reservoir device according to an embodiment.
[Fig. 2] Fig. 2 is a side cross-sectional view illustrating an adapter according to the embodiment.
[Fig. 3] Fig. 3 (a) is a perspective view illustrating a reservoir container according to the embodiment, and (b) is a side view illustrating the adapter.
[Fig. 4] Fig. 4 is a plan view illustrating elements of the adapter excluding a base and a seal member according to the embodiment.
[Fig. 5] Fig. 5 is a plan view illustrating the reservoir container according to the embodiment.
[Fig. 6] Fig. 6 (a) is a view illustrating a state where the adapter and the reservoir container are in a first relative position in an attachment operation, (b) is a view illustrating a state where the adapter and the reservoir container are in a second relative position, and (c) is a view illustrating a state where the adapter and the reservoir container are in a third relative position.
[Fig. 7] Fig. 7 (a) is a plan view of the adapter according to the embodiment, (b) is an A-A cross-sectional view from (a), (c) is an enlarged view of a dotted line area in (b), and (d) is a side cross-sectional view of a portion of a brim-holding claw including a first engagement element.
[Fig. 8] Fig. 8 (a) is a plan view of an adapter according to a first variation, (b) is an A-A cross-sectional view from (a), (c) is an enlarged view of a dotted line area in (b), and (d) is a side cross-sectional view of a portion of a brim-holding claw including a first engagement element.
[Fig. 9] Fig. 9 (a) is a plan view of an adapter according to a second variation, (b) is an A-A cross-sectional view from (a), (c) is an enlarged view of a dotted line area in (b), and (d) is a side cross-sectional view of a portion of a brim-holding claw including a first engagement element.
[Fig. 10] Fig. 10 (a) is a side cross-sectional view of an adapter having a brim-holding claw of a first mode according to a third variation, (b) is a partial side cross-sectional view (a cross-sectional view along the line B-B in Fig. 5) of a brim according to the third variation, (c) is a partial side cross-sectional view of a brim-holding claw of a second mode according to the third variation, and (d) is a partial side cross-sectional view of a brim-holding claw of a third mode according to the third variation.

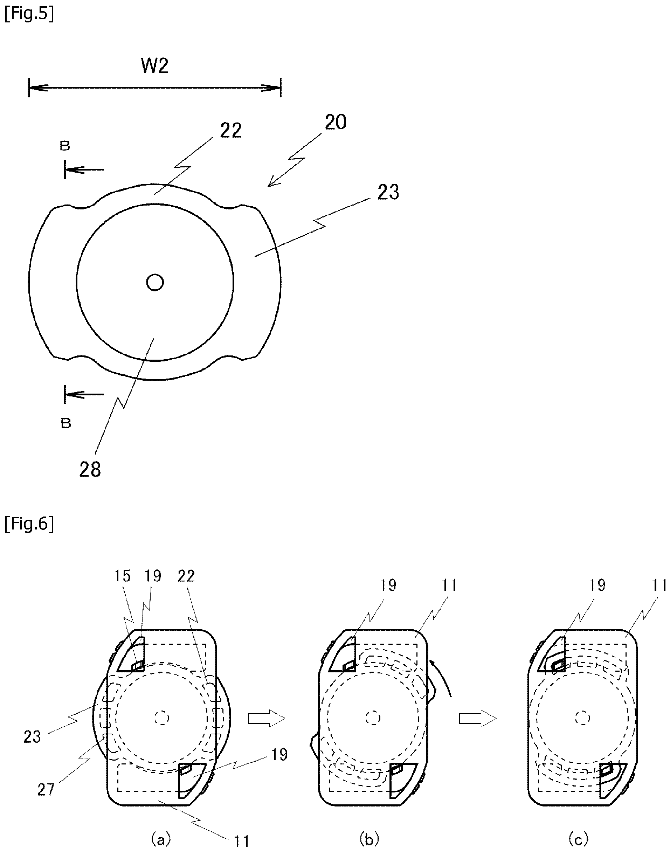

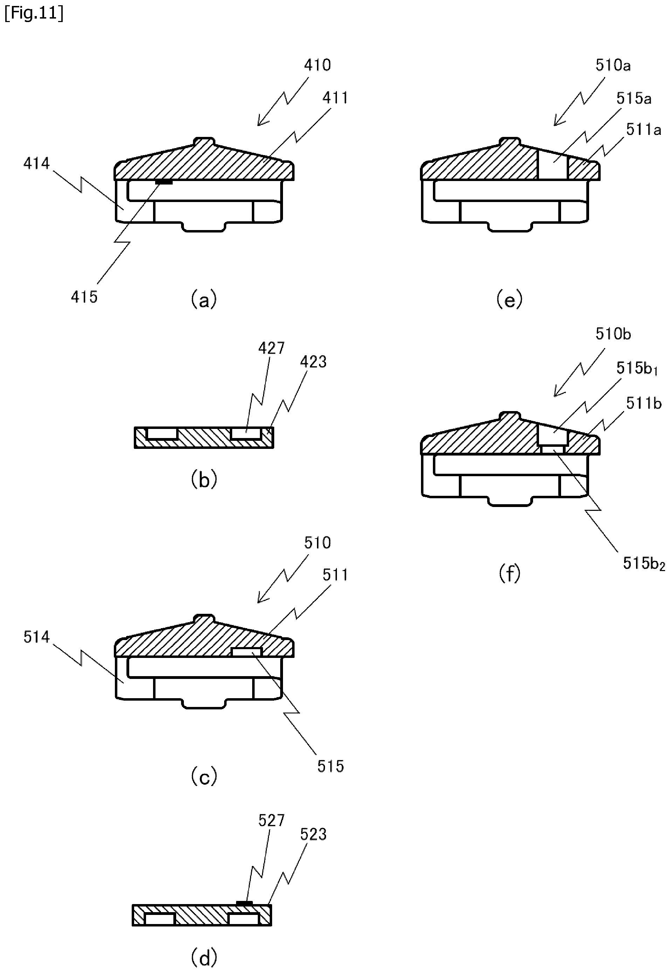

[Fig. 11] Fig. 11 (a) is a side cross-sectional view of an adapter according to a fourth variation, (b) is a partial side cross-sectional view (a cross-sectional view along the line B-B in Fig. 5) of a brim according to the fourth variation, (c) is a side cross-sectional view of an adapter of a first mode according to a fifth variation, (d) is a partial side cross-sectional view (a cross-sectional view along the line B-B in Fig. 5) of a brim according to the fifth variation, (e) is a side cross-sectional view of an adapter of a second mode according to the fifth variation, and (f) is a side cross-sectional view of an adapter of a third mode according to the fifth variation.

### Mode for Carrying out the Invention

Embodiment examples of the present invention will be described below.

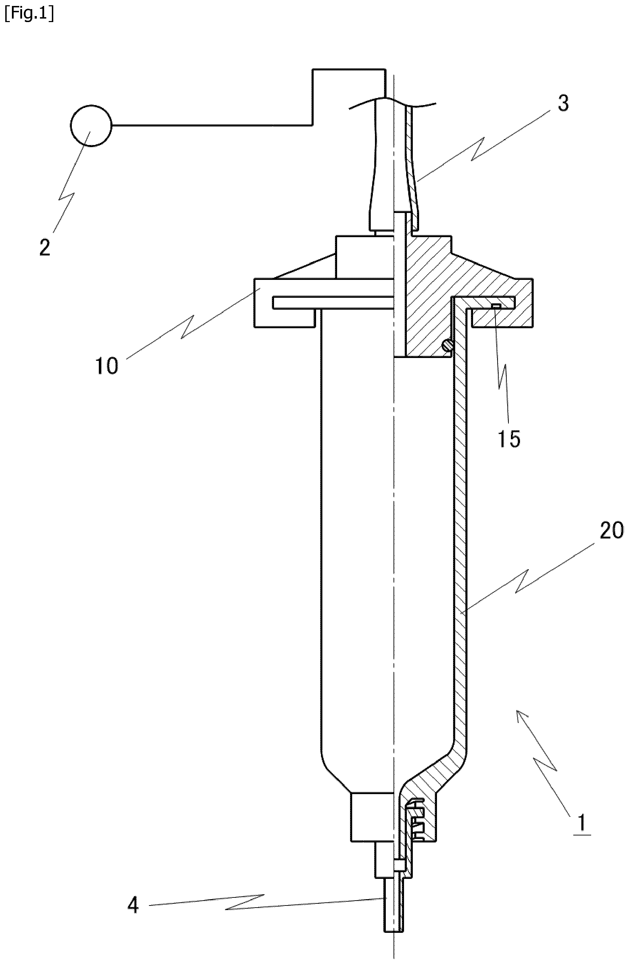

As illustrated in Fig. 1, a reservoir device 1 according to an embodiment includes an adapter 10 and a reservoir container 20. An upper end of the reservoir container 20 is connected to the adapter 10 and a lower end is connected to a nozzle 4. In Fig. 1, for convenience of explanation, a second lateral member 12b to be described later is omitted.

The adapter 10 is connected to a pressurized-air supply source 2 via an air pipe 3, and pressurized air (or gas) is supplied from the pressurized-air supply source 2 to the reservoir container 20.

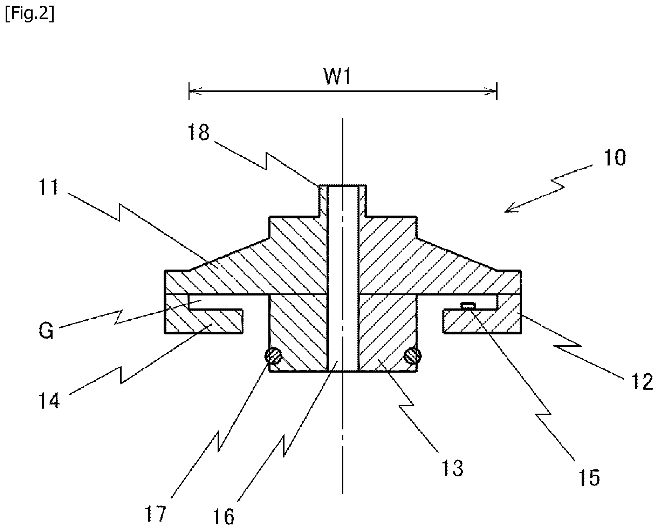

Fig. 2 is a side cross-sectional view illustrating the adapter 10 according to the embodiment. The adapter 10 includes a base 11, a pair of lateral members 12 extending downward from the base 11, a blocking portion 13 extending downward from a central portion of the base 11, a pair of brim-holding claws 14 extending toward the blocking portion 13 from lower ends of the pair of lateral members 12, and first engagement elements 15. In Fig. 2, for convenience of explanation, only one first engagement element 15 is illustrated and the second lateral member 12b to be described later is omitted.

The base 11, the lateral members 12, the blocking portion 13, the brim-holding claws 14, and the first engagement elements 15 that constitute the adapter 10 are integrally molded by injection molding. The adapter 10 according to the embodiment is made of harder resin material than the reservoir container 20 to prevent air leakage due to deformation. From the viewpoint of enhancing visibility in engagement of the first engagement elements 15, it is preferable that the adapter 10 is made of colored resin material and the reservoir container 20 is made of transparent resin as described later.

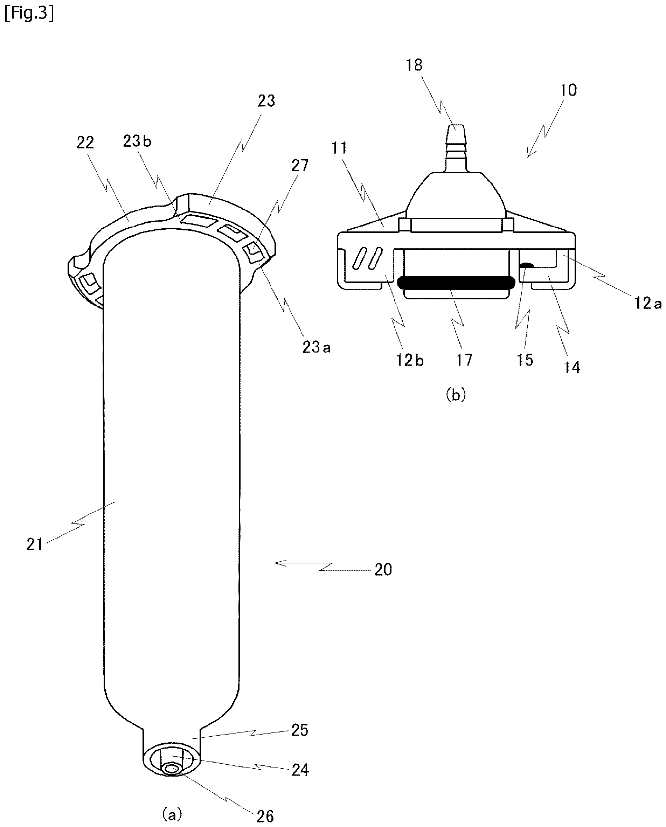

The base 11 has a size that can cover brims 23 of the reservoir container 20 and is provided with a joint 18 extending upward from the central portion as illustrated in Fig. 3 (the joint 18 is simplified in Fig. 2). A bottom surface of the base 11 is provided with the blocking portion 13 at the central portion and the lateral members 12, 12 on both short sides.

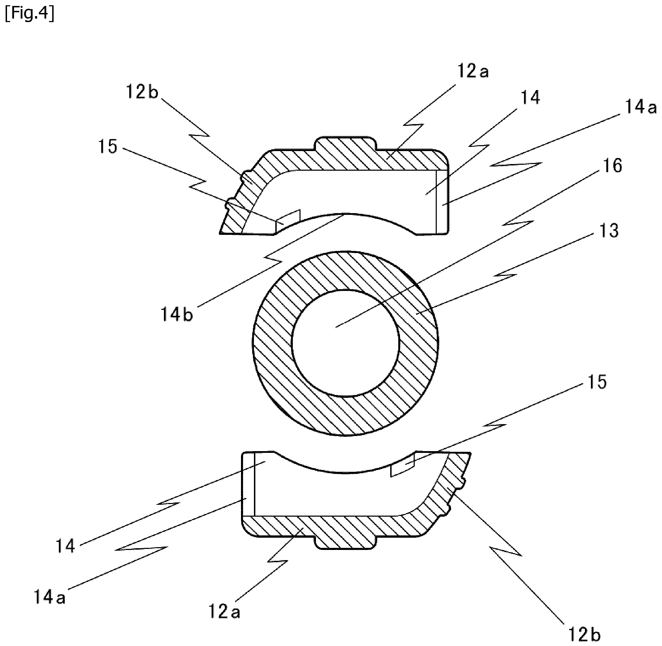

The lateral member 12 is constituted by a first lateral member 12a and a second lateral member 12b extending downward from the base 11 and has a size that can cover a brim 23 (see Figs. 4 and 6 (c) to be described later). From a lower end of the lateral member 12, a plate-shaped brim-holding claw 14 extends substantially horizontally toward the blocking portion 13. The lateral member 12 and the brim-holding claw 14 constitute a brim-holding portion that holds the brim 23. When the adapter 10 is attached, the brim 23 of the reservoir container 20 enters a gap G between the brim-holding claw 14 and the base 11. Although the gap G is smaller than the thickness of the brim 23, when the brim 23 enters the gap G, the gap G widens due to elastic deformation of the brim-holding claw 14. The distance W1 between inner walls of the lateral members 12, 12 is larger than the distance W2 between most protruded points of the brims 23, 23 to be described later.

The blocking portion 13 is a columnar member extending downward from the central portion of the base 11 and is provided with an annular seal member 17 slightly above its lower end. The blocking portion 13 has a through-hole 16, and pressurized air is supplied from the pressurized-air supply source 2 into the reservoir container 20 via the through-hole 16.

The first engagement element 15 is provided on an upper surface of the brim-holding claw 14. The first engagement element 15 is a protruded portion provided at a position corresponding to a second engagement element 27 of the reservoir container 20 to be described later. In the embodiment, although the first engagement element 15 is provided on each of the pair of brim-holding claws 14, it is also conceivable that the first engagement element 15 is provided only on one of the pair of brim-holding claws 14.

Fig. 3 (a) is a perspective view illustrating the reservoir container 20 according to the embodiment, and (b) is a side view illustrating the adapter 10.

The reservoir container 20 is a syringe including a cylindrical container body 21 having an upper opening at its end, an annular flange 22 around the edge of the upper opening 28 of the container body 21, a pair of brims 23, 23 extending radially outward from the flange 22, an inner cylinder 24 having a lower opening smaller in diameter than the upper opening 28, and an outer cylinder 25 of which inner surface is provided with a groove for screwing in the nozzle 4. As illustrated in Fig. 5, the pair of brims 23, 23 are arranged at opposite positions symmetrically about the center of the upper opening 28 when viewed from above. The distance W2 between the most protruded point of a first brim 23 and the most protruded point of a second brim 23 (see Fig. 5) is smaller than the distance W1 between the inner walls of the lateral members 12, 12 (see Fig. 2). With this configuration, as the most protruded points of the brims 23 are always away from the inner walls of the lateral members 12, 12 during an attachment operation of the adapter 10, rotation becomes easier.

A second engagement element 27 that is a recessed portion is provided in a lower surface of each of the brims 23, 23 according to the embodiment. In the example of Fig. 3 (a), three recessed portions are provided in the lower surface of the brim 23. In this embodiment, the reservoir container 20 is rotated counterclockwise when viewed from the adapter 10 side so that the first engagement element 15 is engaged with the second engagement element 27. In such a configuration, the recessed portion adjacent to a lower-surface outer short side 23a present in the forward movement direction serves as the second engagement element. Unlike this, in a configuration where the reservoir container 20 is rotated clockwise when viewed from the adapter 10 side so that the first engagement element 15 is engaged with the second engagement element 27, the recessed portion adjacent to a lower-surface outer short side 23b present in the forward movement direction serves as the second engagement element. The second engagement element 27 is large enough to accommodate the first engagement element 15 and has such a size that at least a part of an outer surface of the first engagement element 15 comes in contact with a part of an inner surface of the second engagement element 27. The second engagement element 27 may be configured by utilizing a plurality of the recessed portions provided in the lower surface of the brim of the reservoir container for preventing deformation and the like.

Since the reservoir container 20 according to the embodiment is made of transparent resin, the first engagement element 15 is visible through the second engagement element 27. Although the reservoir container 20 can be made of transparent resin (for example, polypropylene (PP)), in a case where UV curing adhesive or the like is stored, the reservoir container 20 is made of light-shielding resin.

Fig. 4 is a plan view illustrating elements of the adapter 10 excluding the base 11 and the seal member 17 according to the embodiment.

The lateral members 12 extending downward from both the short sides of the base 11 are each constituted by the first lateral member 12a extending in a first direction and the second lateral member 12b extending in a second direction intersecting with the first direction, and the second lateral member 12b serves as a rotation stop surface that comes in contact with a side surface of the brim 23.

A tapered surface 14a is formed on a short side on the first lateral member side of the brim-holding claw 14. The tapered surface 14a acts to smoothly receive the brim 23 into the gap G when the adapter 10 is inserted and fixed into the reservoir container 20. Hereinafter, the portion within the gap G adjacent to the tapered surface 14a is sometimes referred to as near a brim entrance start position. When rotation is performed with the blocking portion 13 inserted into the upper opening 28 of the reservoir container 20, the brim 23 which has entered the gap G from the portion of the tapered surface 14a comes in contact with the rotation stop surface (second lateral member 12b) located in the forward movement direction to stop the rotation. Hereinafter, the portion within the gap G adjacent to the second lateral member 12b is sometimes referred to as near an innermost point. Note that it is not necessary to provide the lateral member 12 with the rotation stop surface, and the lateral member 12 may be constituted only by the first lateral member 12a without the second lateral member 12b.

An inner periphery 14b of the brim-holding claw 14 has an arc shape slightly larger than the arc of the flange 22 of the reservoir container 20. The respective upper surfaces of the pair of brim-holding claws 14, 14 (near the innermost points of the gaps) are provided with the first engagement elements 15, 15 at positions symmetrical about the blocking portion 13. The first engagement elements 15, 15 are adapted to be engaged with the second engagement elements 27, 27 in such a position that short sides of the brims 23 come in contact with the rotation stop surfaces to stop the rotation. From another perspective, the first engagement element 15 is arranged at such a position that it is in contact with the lower-surface outer short side 23a of the brim 23 in a state where a large part of the turned brim 23 is in the gap G (a state where the brim 23 has been rotated more than 45 degrees from the initial position). With this configuration, the duration of experiencing resistance by the first engagement element 15 can be minimized during an operation of turning the brim 23 of the reservoir container 20 within the gap G, and attrition of the first engagement element 15 caused by sliding of the brim 23 can be minimized.

Although the first engagement element 15 is a protruded portion that is substantially parallelogram shaped when viewed from above, the shape is not limited to the illustrated one and a protruded portion of any shape is possible.

Fig. 6 (a) is a view illustrating a state where the adapter 10 and the reservoir container 20 are in a first relative position in the attachment operation, (b) is a view illustrating a state where the adapter 10 and the reservoir container 20 are in a second relative position, and (c) is a view illustrating a state where the adapter 10 and the reservoir container 20 are in a third relative position. In Fig. 6, for convenience of explanation, the base 11 is partially omitted and is depicted transparently.

As illustrated in Fig. 6 (a), the adapter 10 is placed in a positional relationship where the short sides of the base 11 do not overlap the brims 23 of the reservoir container 20 (for example, a positional relationship where the long sides of the adapter 10 are substantially perpendicular to the long sides of the reservoir container 20), and the blocking portion 13 is inserted into the upper opening 28 of the reservoir container 20. In the positional relationship of Fig. 6 (a), the brims 23 of the reservoir container 20 are located between the tapered surfaces 14a of the brim-holding claws and the second lateral members 12b of the brim-holding claws. In this situation, when trying to rotate the reservoir container 20 clockwise with respect to the adapter 10, the brims 23 come in contact with the second lateral members 12b, and the rotation of the reservoir container 20 is restricted (see Fig. 4). In the positional relationship of Fig. 6(a), the first engagement elements 15 are visible through windows 19.

As illustrated in Fig. 6 (b), when the reservoir container 20 is rotated counterclockwise with respect to the adapter 10, the brims 23 of the reservoir container 20 enter the gaps G. Since the entrance portions within the gaps G have the tapered surfaces 14a, the brims 23 smoothly enter the gaps G. Further, when the brims 23 enter the gaps G, the gaps G widen due to elastic deformation of the brim-holding claws 14, allowing smooth rotational movement of the reservoir container 20. In addition, during the rotational movement of the reservoir container 20, the most protruded points of the brims 23, 23 are not in contact with the inner walls of the lateral members 12, 12 (in the relationship of W1 > W2), which also contributes to the smoothness of the rotational movement.

As illustrated in Fig. 6 (c), when the reservoir container 20 is further rotated, the short sides of the brims 23 come in contact with the rotation stop surfaces to stop the rotation of the reservoir container 20. At this point, the first engagement elements 15 are engaged with the second engagement elements 27 and a clicking sensation can be felt by hand. Preferably, a clicking sound is made simultaneously with the engagement of the first engagement elements 15 with the second engagement elements 27. This allows an operator to figure out through auditory and haptic senses that the adapter 10 has been fixed to the reservoir container 20. Further, it is also possible to confirm that the adapter 10 and the reservoir container 20 are in the right connection position by viewing the first engagement elements 15 through the windows 19.

Although the attachment method is illustrated in Fig. 6 in which the reservoir container 20 is rotated, attachment may be performed by rotating the adapter 10 clockwise when viewed from above while the reservoir container 20 is kept stationary.

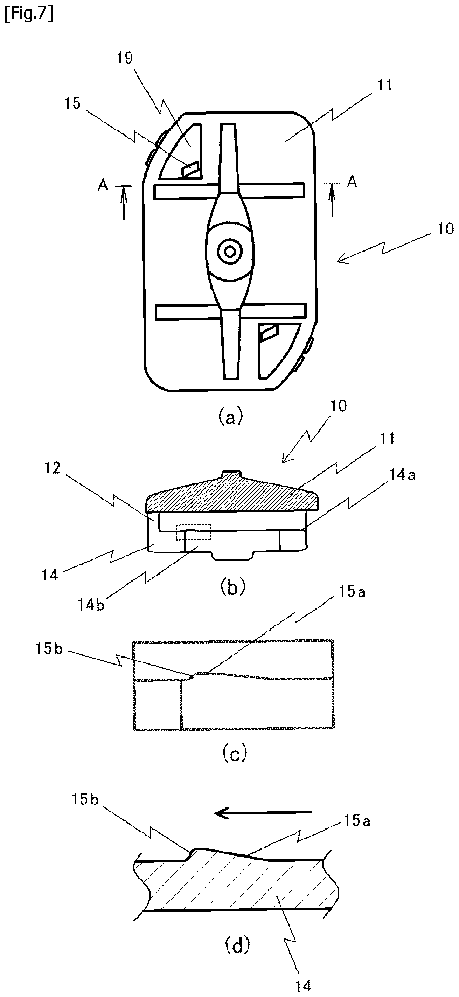

Fig. 7 (a) is a plan view of the adapter 10 according to the embodiment, (b) is an A-A cross-sectional view from (a), (c) is an enlarged view of a dotted line area in (b), and (d) is a side cross-sectional view of a portion of the brim-holding claw 14 including a first engagement element 15.

As illustrated in Fig. 7 (a), the adapter 10 has a substantially rectangular shape with opposite two corners rounded when viewed from above, and the base 11 is provided with the windows 19 near the rounded corners. The first engagement elements 15 are visible through the windows 19. The window 19 is formed by extracting a mold (insert) for forming the first engagement element when the adapter 10 is injection molded. In a case where no window 19 is provided, there is an issue that the protruded portion of the first engagement element 15 stands in the way and prevents the mold from being extracted. If the mold is forced to be extracted, there may arise an issue that the protruded portion is peeled back or crushed. From another perspective, providing the windows 19 achieves an increased freedom in shape of protruded portions of the first engagement elements 15.

In a case where first engagement elements 15 are provided on a lower surface of the base 11, windows 19 are preferably provided in the brim-holding claws 14.

As illustrated in Fig. 7 (b), the first engagement element 15 is a protruded portion formed on the upper surface of the brim-holding claw 14.

As illustrated in Fig. 7 (c) and (d), the first engagement element 15 has a first slope 15a on a side where the brim 23 first comes into contact and a second slope 15b on the other side across the apex. The first slope 15a is gentler than the second slope 15b, which makes it easier for the brim 23 to pass over the apex of the first engagement element. The brim 23 elastically deforms in an upward warping manner when passing over the first engagement element 15. When the second engagement element 27 engages with the first engagement element 15, the brim 23 that has upward warped reverts and a clicking sensation is transmitted to the hand of the operator. In the configuration example of Fig. 7, the second slope 15b is steeper than the first slope 15a, which causes a clear clicking sensation when the first engagement element 15 is engaged with the second engagement element 27.

The first engagement element 15 can be a protruded portion of any shape.

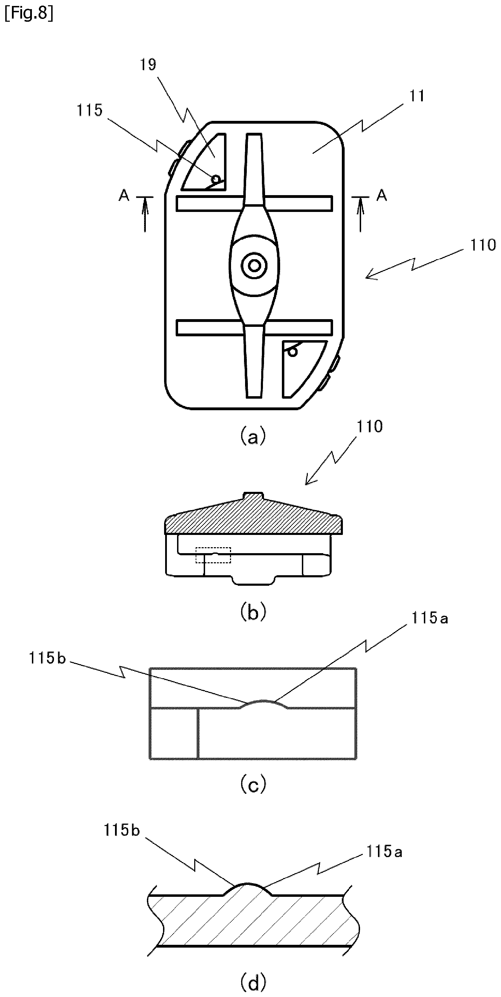

Fig. 8 (a) is a plan view of an adapter 110 according to a first variation, (b) is an A-A cross-sectional view from (a), (c) is an enlarged view of a dotted line area in (b), and (d) is a side cross-sectional view of a portion of a brim-holding claw including a first engagement element 115. The first engagement element 115 according to the first variation has a semicircular cross-section, and includes a first slope 115a and a second slope 115b having symmetrical shapes. This causes more force to be required for engagement of the first engagement element 115 with the second engagement element (not illustrated) and a clicking sensation obtained upon the engagement to be milder than in the embodiment.

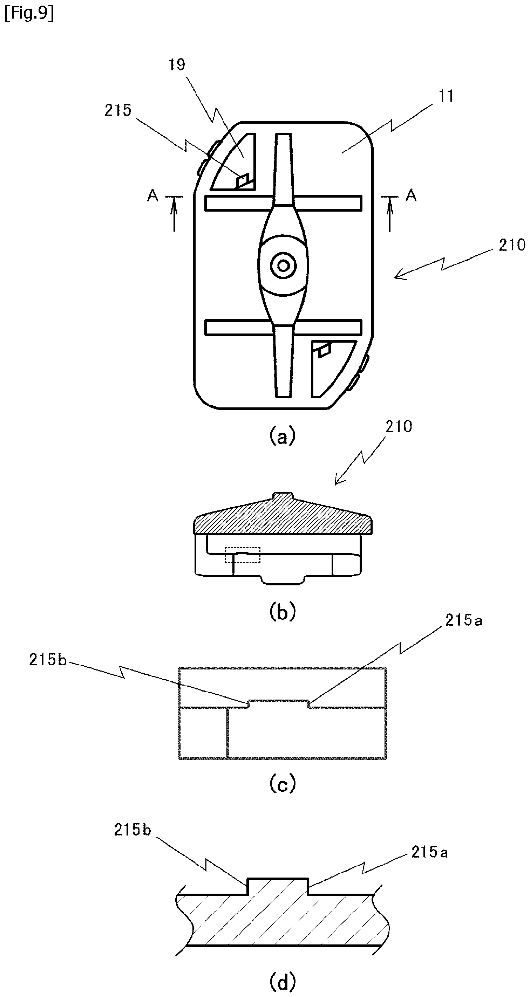

Fig. 9 (a) is a plan view of an adapter 210 according to a second variation, (b) is an A-A cross-sectional view from (a), (c) is an enlarged view of a dotted line area in (b), and (d) is a side cross-sectional view of a portion of a brim-holding claw including a first engagement element 215. The first engagement element 215 according to the second variation has a rectangular cross-section. In the adapter 210 according to the second variation, the first engagement element 215 includes a first face 215a and a second face 215b that are parallel to the vertical direction. This causes more force to be required for engagement of the first engagement element 215 with the second engagement element (not illustrated) and a clicking sensation obtained upon the engagement to be stronger than in the first variation. Both in the first and second variations, windows 19 are provided and thus the issue of extracting a mold does not arise, which achieves an increased freedom in shape of protruded portions of the first engagement elements 15.

The technical idea of the present invention can be realized even with a combination of a first engagement element that is a recessed portion and a second engagement element that is a protruded portion unlike the examples described above.

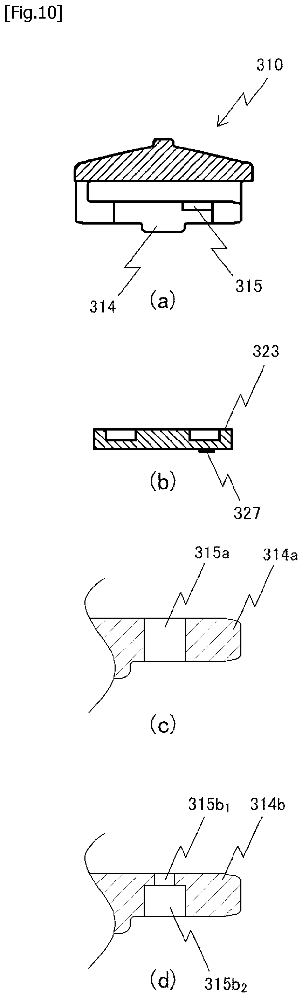

Fig. 10 (a) is a side cross-sectional view of an adapter 310 having a brim-holding claw 314 of a first mode according to a third variation, (b) is a side cross-sectional view of a brim 323 according to the third variation, (c) is a partial side cross-sectional view of a brim-holding claw 314a of a second mode according to the third variation, and (d) is a partial side cross-sectional view of a brim-holding claw 314b of a third mode according to the third variation.

As illustrated in Fig. 10 (a), a first engagement element 315 of the first mode is a recessed portion formed in the upper surface of the brim-holding claw 314. As illustrated in Fig. 10 (b), a second engagement element 327 is a protruded portion formed on the lower surface of the brim 323.

The recessed portion of the first engagement element 315 is of a size that allows engagement with the protruded portion of the second engagement element 327. Herein, a size that allows engagement with the protruded portion means a size that allows accommodation of the protruded portion. The first engagement element 315 and the second engagement element 327 are arranged at such positions that they engage with each other near the brim entrance start position of a gap G. Note that the recessed portion of the first engagement element 315 does not need to have a rectangular cross-section as illustrated, and may have a cross-section of square shape, trapezoidal shape, semicircular shape, semi-elliptical shape, or the like. The same applies to the second engagement element 327.

As illustrated in Fig. 10 (c), the brim-holding claw 314a of the second mode is provided with a first engagement element 315a that is a constant-diameter hole penetrating the brim-holding claw 314a. As illustrated in Fig. 10 (d), the brim-holding claw 314b of the third mode is provided with a first engagement element that is a stepped hole (an upper small-diameter hole 315b₁ and a lower large-diameter hole 315b₂) penetrating the brim-holding claw 314b. The first engagement element 315a, 315b₁, 315b₂ is engaged with the second engagement element 327 that is a protruded portion formed on the lower surface of the brim 323 (see Fig. 10 (b)) near the brim entrance start position of the gap G. In this way, a hole penetrating the brim-holding claw of the adapter may form a first engagement element that is a recessed portion. The first engagement element 315a, 315b₁, 315b₂ can be easily formed by placing a mold (insert) for forming the first engagement element. Note that the first engagement element 315a, 315b₁, 315b₂ is not limited to a circular-shaped hole when viewed from above, and may be a hole of elliptical shape, polygonal shape, or the like when viewed from above.

Alternatively, a first engagement element may be provided on the lower surface of the base of the adapter instead of the brim-holding claw. However, the first engagement element needs to be provided in such a place that it is possible to utilize the behavior of the brim of the reservoir container easily elastically deforming in the vertical direction.

Fig. 11 (a) is a side cross-sectional view of an adapter 410 according to a fourth variation including a first engagement element 415 that is a protruded portion provided on the lower surface of a base 411, and (b) is a side cross-sectional view (a cross-sectional view along the line B-B in Fig. 5) of a brim 423 according to the fourth variation including a second engagement element 427 that is a recessed portion provided in the upper surface of the brim 423.

The protruded portion of the first engagement element 415 is of a size that allows engagement with the recessed portion of the second engagement element 427 (including a size smaller than that of the recessed portion). The first engagement element 415 and the second engagement element 427 are arranged at such positions that they engage with each other near the innermost point of a gap G. Note that the protruded portion of the first engagement element 415 does not need to have a rectangular cross-section as illustrated, and may have a cross-section of square shape, trapezoidal shape, semicircular shape, semi-elliptical shape, or the like. The same applies to the second engagement element 427.

Fig. 11 (c) is a side cross-sectional view of an adapter 510 of a first mode according to a fifth variation including a first engagement element 515 that is a recessed portion provided in the lower surface of a base 511, and (d) is a side cross-sectional view (a cross-sectional view along the line B-B in Fig. 5) of a brim 523 according to the fifth variation including a second engagement element 527 that is a protruded portion provided on the upper surface of the brim 523.

In the adapter 510 of the first mode, the recessed portion of the first engagement element 515 is of a size that allows engagement with the protruded portion of the second engagement element 527 (a size that allows accommodation of the protruded portion). The first engagement element 515 and the second engagement element 527 are arranged at such positions that they engage with each other near the brim entrance start position of a gap G. Note that the recessed portion of the first engagement element 515 does not need to have a rectangular cross-section as illustrated, and may have a cross-section of square shape, trapezoidal shape, semicircular shape, semi-elliptical shape, or the like. The same applies to the second engagement element 527.

When an adapter of Fig. 11 (a) and (c) is injection molded, a brim-holding claw 414, 514 is desirably provided with an opening at a position facing the first engagement element 415, 515. The reasons for this are that it is possible to enhance the visibility of the first engagement element 415, 515 and that it is necessary to place a mold (insert) for forming the first engagement element 415, 515.

Fig. 11 (e) is a side cross-sectional view of an adapter 510a of a second mode according to the fifth variation, and (f) is a side cross-sectional view of an adapter 510b of a third mode according to the fifth variation.

In the adapter 510a of the second mode, a first engagement element 515a is a constant-diameter hole penetrating a base 511a. In the adapter 510b of the third mode, a first engagement element is a stepped hole (an upper large-diameter hole 515b₁ and a lower small-diameter hole 515b₂) penetrating a base 511b. The first engagement element 515a, 515b₁, 515b₂ is engaged with the second engagement element 527 that is a protruded portion formed on the upper surface of the brim 523 (see Fig. 11 (d)) near the brim entrance start position of a gap G. In this way, a hole penetrating the base of the adapter may form a first engagement element that is a recessed portion. The first engagement element 515a, 515b₁, 515b₂ can be easily formed by placing a mold (insert) for forming the first engagement element. Note that the first engagement element 515a, 515b₁, 515b₂ is not limited to a circular-shaped hole when viewed from above, and may be a hole of elliptical shape, polygonal shape, or the like when viewed from above.

Combination examples of first engagement elements and second engagement elements described above are summarized in following Table 1.

**[Table 1]**

| | First Engagement Element | Second Engagement Element | | | | |
|---|---|---|---|---|---|---|
| (1) | Brim-Holding Claw | Upper Surface | Protruded | Brim | Lower Surface | Recessed |
| (2) | Brim-Holding Claw | Upper Surface | Recessed | Brim | Lower Surface | Protruded |
| (3) | Base | Lower Surface | Protruded | Brim | Upper Surface | Recessed |
| (4) | Base | Lower Surface | Recessed | Brim | Upper Surface | Protruded |

In either combination of Table 1, the behavior of the brim-holding claw of the adapter easily elastically deforming in the vertical direction is utilized. Therefore, even if some dimensional difference occurs due to uneven shrinkage during manufacturing, the first engagement element can be appropriately engaged with the second engagement element. In either combination of Table 1, each engagement element is preferably arranged at such a position that the timing when resistance is given by the first engagement element during turning the brim of the reservoir container within the gap G can be delayed and attrition caused by sliding of the brim can be minimized. In a case where the first engagement element is protruded and the second engagement element is recessed, it is preferable that the first engagement element and the second engagement element engage with each other near the innermost point of the gap G when the reservoir container or the adapter has been rotated to make the brim enter the gap G. Specifically, the first engagement element is preferably provided near the furthest side from the brim entrance side of the gap G (in other words, near the second lateral member 12b), and the second engagement element is preferably provided near the side where the brim starts entering the gap G. In a case where the first engagement element is recessed and the second engagement element is protruded, it is preferable that the first engagement element and the second engagement element engage with each other near the brim entrance start position of the gap G when the reservoir container or the adapter has been rotated to make the brim enter the gap G. Specifically, the first engagement element is preferably provided near the brim entrance start side of the gap G (in other words, near the furthest side from the second lateral member 12b), and the second engagement element is preferably provided near the furthest side from the side where the brim starts entering the gap G.

From another perspective, in a case where the first engagement element is protruded and the second engagement element is recessed, the first engagement element is preferably arranged at a position on a side closer to the innermost point of the gap G than a virtual line extending in the longitudinal direction of the adapter and passing through the center of the blocking portion 13 when the adapter is viewed from above (more preferably, a side closer to the innermost point of the gap G such that the angle between that virtual line and a line connecting the first engagement element and the center of the blocking portion 13 is 10 degrees or more), and the second engagement element is preferably arranged at a position which is closer to the short side of the brim that enters the gap G first (in a case where the reservoir container is rotated counterclockwise when viewed from above as in the configuration example of Fig. 3, the short side 23a) than a virtual line extending in the longitudinal direction of the brim and passing through the center of the upper opening 28 when the reservoir container is viewed from above (more preferably, such a position that the angle between that virtual line and a line connecting the second engagement element and the center of the upper opening 28 is 10 degrees or more). In a case where the first engagement element is recessed and the second engagement element is protruded, the first engagement element is preferably arranged at a position on a side closer to the brim entrance start position of the gap G than a virtual line extending in the longitudinal direction of the adapter and passing through the center of the blocking portion 13 when the adapter is viewed from above (more preferably, a side closer to the brim entrance start position such that the angle between that virtual line and a line connecting the first engagement element and the center of the blocking portion 13 is 10 degrees or more), and the second engagement element is preferably arranged at a position which is closer to the short side of the brim opposite to the short side that enters the gap G first (in a case where the reservoir container is rotated counterclockwise when viewed from above as in the configuration example of Fig. 3, the short side 23b) than a virtual line extending in the longitudinal direction of the brim and passing through the center of the upper opening 28 when the reservoir container is viewed from above (more preferably, such a position that the angle between that virtual line and a line connecting the second engagement element and the center of the upper opening 28 is 10 degrees or more).

In either combination of Table 1, since a clicking sensation can be felt by hand upon engagement of the first engagement element with the second engagement element, it is possible to easily confirm that the reservoir container and the adapter are in the right connection position. It is more preferable that a clicking sound is made simultaneously with the clicking sensation.

Moreover, since the distance W1 between the inner walls of the lateral members of the adapter is larger than the distance W2 between the most protruded points of the pair of brims, even if some dimensional difference occurs due to uneven shrinkage during manufacturing, no friction resistance between the most protruded points of the brims and the inner walls of the lateral members affects the fit.

Furthermore, by providing the window through which the first engagement element is visible, it is possible to visually confirm that the reservoir container and the adapter are in the right connection position. When the reservoir container is made of transparent resin and the adapter is made of colored resin, the visibility of the first engagement element is further enhanced. In addition, by arranging a mold (insert) for forming the first engagement element when the adapter is injection molded, freedom in shape of the first engagement element can be enhanced.

Although the adapter is replaced when it is dirty or damaged, considering that several reservoir containers (syringes) are repeatedly attached to and detached from the adapter until the replacement, the adapter is preferably made of harder material than the reservoir container.

Although preferred embodiment examples of the present invention have been described above, the technical scope of the present invention is not limited to the descriptions of the above embodiment examples. Various alterations and modifications can be applied without departing from the technical idea of the present invention, and such altered or modified modes also fall within the technical scope of the present invention.

For example, also in metal casting, deformation sometimes occurs due to so-called shrinkage cavities (cavities caused by solidification shrinkage). Therefore, the technical idea of the present invention can also be applied to a reservoir container adapter made of metal.

### List of Reference Symbols

1: reservoir device / 2: pressurized-air supply source / 3: air pipe / 4: nozzle / 10: adapter / 11: base / 12: lateral member / 13: blocking portion / 14: brim-holding claw / 15: first engagement element / 16: through-hole / 17: seal member / 18: joint / 19: window / 20: reservoir container / 21: container body (reservoir cylinder) / 22: flange / 23: brim / 24: inner cylinder / 25: outer cylinder / 26: lower opening / 27: second engagement element/ 28: upper opening / 110, 210, 310, 410, 510: adapter / 115, 215, 315, 415, 515: first engagement element / 327, 427, 527: second engagement element

## Claims

1. A reservoir device comprising a reservoir container and an adapter configured to be attached to the reservoir container,
wherein the reservoir container includes a reservoir cylinder having an upper opening and a lower opening, and a pair of brims extending laterally from an upper end of the reservoir cylinder,
the adapter includes a base configured to be connected to an air pipe, a blocking portion extending downward from a central portion of the base, and a pair of brim-holding portions provided on opposite short sides of the base and configured to hold the pair of brims,
the pair of brim-holding portions include a pair of lateral members extending downward from the base, and a pair of brim-holding claws extending toward the blocking portion from the pair of lateral members,
an upper surface of at least one of the pair of brim-holding claws or a lower surface of the base is provided with a first engagement element, and
a lower surface or an upper surface of each of the pair of brims is provided with a second engagement element configured to engage with the first engagement element,
wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

2. The reservoir device according to claim 1, wherein a distance W1 between inner walls of the pair of lateral members is larger than a distance W2 between most protruded points of the pair of brims.

3. The reservoir device according to claim 1, wherein the base or the brim-holding claws include a window provided at a position facing the first engagement element.

4. The reservoir device according to claim 1, wherein the pair of lateral members include rotation stop surfaces that come in contact with short sides of the pair of brims in such a position that the first engagement element and the second engagement element engage with each other.

5. The reservoir device according to claim 1, wherein the pair of brim-holding claws include tapered surfaces for the brims to come in contact with when entering the gaps between the brim-holding claws and the base.

6. The reservoir device according to claim 1, wherein the gaps between the brim-holding claws and the base have such a size that the holding claws elastically deform when the brims enter the gaps.

7. The reservoir device according to claim 1, wherein the adapter is made of resin.

8. The reservoir device according to claim 1, wherein the reservoir container is made of resin that is transparent, and
the adapter is made of resin that is colored.

9. The reservoir device according to claim 1, wherein the adapter is made of material harder than material of the brims.

10. The reservoir device according to any one of claims 1 to 9, wherein the first engagement element includes a protruded portion provided on an upper surface of each of the pair of brim-holding claws, and
the second engagement element includes a recessed portion provided in a lower surface of each of the pair of brims.

11. The reservoir device according to claim 10, wherein the first engagement element and the second engagement element engage with each other at a position closer to a position furthest from an entrance start position for the brims in the gaps than a midpoint between the entrance start position for the brims in the gaps and the position furthest from the entrance start position for the brims in the gaps.

12. The reservoir device according to any one of claims 1 to 8, wherein the first engagement element includes a recessed portion provided in an upper surface of each of the pair of brim-holding claws, and
the second engagement element includes a protruded portion provided on a lower surface of each of the pair of brims.

13. The reservoir device according to claim 12, wherein the first engagement element and the second engagement element engage with each other at a position closer to an entrance start position for the brims in the gaps than a midpoint between the entrance start position for the brims in the gaps and a position furthest from the entrance start position for the brims in the gaps.

14. The reservoir device according to any one of claims 1 to 9, wherein the first engagement element includes a protruded portion provided on a lower surface of the base, and
the second engagement element includes a recessed portion provided in an upper surface of each of the pair of brims.

15. The reservoir device according to claim 14, wherein the first engagement element and the second engagement element engage with each other at a position closer to a position furthest from an entrance start position for the brims in the gaps than a midpoint between the entrance start position for the brims in the gaps and the position furthest from the entrance start position for the brims in the gaps.

16. The reservoir device according to any one of claims 1 to 8, wherein the first engagement element includes a recessed portion provided in a lower surface of the base, and
the second engagement element includes a protruded portion provided on an upper surface of each of the pair of brims.

17. The reservoir device according to claim 16, wherein the first engagement element and the second engagement element engage with each other at a position closer to an entrance start position for the brims in the gaps than a midpoint between the entrance start position for the brims in the gaps and a position furthest from the entrance start position for the brims in the gaps.

18. A reservoir container adapter used for the reservoir device according to any one of claims 1 to 7, comprising:
a base configured to be connected to an air pipe, a blocking portion extending downward from a central portion of the base, and a pair of brim-holding portions provided on opposite short sides of the base and configured to hold the pair of brims,
wherein the first engagement element includes a protruded portion provided on an upper surface of each of the pair of brim-holding claws,
wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

19. A reservoir container adapter used for the reservoir device according to any one of claims 1 to 7, comprising:
a base configured to be connected to an air pipe, a blocking portion extending downward from a central portion of the base, and a pair of brim-holding portions provided on opposite short sides of the base and configured to hold the pair of brims,
wherein the first engagement element includes a recessed portion provided in an upper surface of each of the pair of brim-holding claws,
wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

20. A reservoir container adapter used for the reservoir device according to any one of claims 1 to 7, comprising:
a base configured to be connected to an air pipe, a blocking portion extending downward from a central portion of the base, and a pair of brim-holding portions provided on opposite short sides of the base and configured to hold the pair of brims,
wherein the first engagement element includes a protruded portion provided on a lower surface of the base,
wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

21. A reservoir container adapter used for the reservoir device according to any one of claims 1 to 7, comprising:
a base configured to be connected to an air pipe, a blocking portion extending downward from a central portion of the base, and a pair of brim-holding portions provided on opposite short sides of the base and configured to hold the pair of brims,
wherein the first engagement element includes a recessed portion provided in a lower surface of the base,
wherein, when the blocking portion is inserted into the upper opening of the reservoir container and the reservoir container is rotated in one direction, the brims enter gaps between the brim-holding claws and the base and the first engagement element and the second engagement element engage with each other to perform positioning.

22. A reservoir container used for the reservoir device according to any one of claims 1 to 6,
the reservoir container comprising a reservoir cylinder having an upper opening and a lower opening, and a pair of brims extending laterally from an upper end of the reservoir cylinder,
wherein the second engagement element includes a recessed portion provided in a lower surface of each of the pair of brims,
wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

23. A reservoir container used for the reservoir device according to any one of claims 1 to 6,
the reservoir container comprising a reservoir cylinder having an upper opening and a lower opening, and a pair of brims extending laterally from an upper end of the reservoir cylinder,
wherein the second engagement element includes a protruded portion provided on a lower surface of each of the pair of brims,
wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

24. A reservoir container used for the reservoir device according to any one of claims 1 to 6,
the reservoir container comprising a reservoir cylinder having an upper opening and a lower opening, and a pair of brims extending laterally from an upper end of the reservoir cylinder,
wherein the second engagement element includes a recessed portion provided in an upper surface of each of the pair of brims,
wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.

25. A reservoir container used for the reservoir device according to any one of claims 1 to 6,
the reservoir container comprising a reservoir cylinder having an upper opening and a lower opening, and a pair of brims extending laterally from an upper end of the reservoir cylinder,
wherein the second engagement element includes a protruded portion provided on an upper surface of each of the pair of brims,
wherein, when the blocking portion of the adapter is inserted into the upper opening of the reservoir container and the adapter is rotated in one direction, the brims enter the gaps between the brim-holding claws and the base and the first engagement element and the second engagement element engage with each other to perform positioning.
